(19) Europäisches Patentamt
European Patent Office
Office européen des brevets

(11) Publication number: **0 047 620 B1**

(12) # EUROPEAN PATENT SPECIFICATION

(45) Date of publication of patent specification: **13.03.85**

(21) Application number: **81303976.5**

(22) Date of filing: **01.09.81**

(51) Int. Cl.$^4$: **C 07 D 487/04,** A 61 K 31/50 // (C07D487/04, 237/00, 237/00),(C07D487/04, 237/00, 231/00)

(54) **Aryl diazabicyclyl amides, a process for their preparation and use.**

(30) Priority: **10.09.80 GB 8029195**

(43) Date of publication of application: **17.03.82 Bulletin 82/11**

(45) Publication of the grant of the patent: **13.03.85 Bulletin 85/11**

(84) Designated Contracting States: **BE CH DE FR IT LI NL**

(56) References cited:
**DE-A-2 748 260**
**DE-A-2 816 884**

**A. BURGER; Med.Chem. Part I, New York, London (1970), page 718**

(73) Proprietor: **BEECHAM GROUP PLC**
**Beecham House Great West Road**
**Brentford Middlesex TW8 9BD (GB)**

(72) Inventor: **Hadley, Michael Stewart**
**9 Coney Gree**
**Sawbridgeworth Hertfordshire (GB)**
Inventor: **King, Francis David**
**67 Cherry Garden Lane**
**Newport Essex (GB)**

(74) Representative: **Stott, Michael John et al**
**European Patent Attorney Beecham Pharmaceuticals Great Burgh Yew Tree Bottom Road**
**Epsom Surrey KT18 5XQ (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 047 620 B1

**Description**

This invention relates to a group of novel compounds, their formulation as pharmaceutical compositions, and their use in the therapy of disorders.

West German Offenlegungsschrift No: 2,748,260.6 describes compounds of the formula (A), and the pharmaceutically acceptable salts thereof:

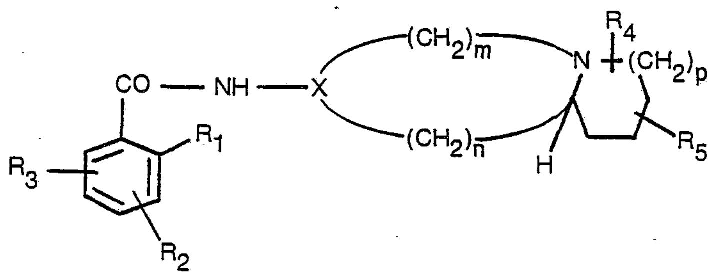

(A)

wherein

$R_1$ is a $C_{1-6}$ alkoxy group;

$R_2$ and $R_3$ are the same or different and are hydrogen, halogen, $CF_3$, hydroxy, $C_{1-6}$ alkoxy, $C_{2-7}$ acyl, amino, amino subsituted by one or two $C_{1-6}$ alkyl groups, $C_{2-7}$ acyl amino, aminocarbonyl or aminosulphone optionally substituted by one or two $C_{1-6}$ alkyl groups, $C_{1-6}$ alkylsulphone or nitro groups;

X is either a nitrogen atom, in which case m + n is 3 to 5, m is 2 to 4 and n is 1 to 3; or X is CH in which case m + n is 2 to 5, m is 1 to 5, and n is 0 to 4;

p is 0 to 3; and

$R_4$ is hydrogen, $C_{1-6}$ alkyl, phenyl or phenyl-$C_{1-6}$ alkyl, either of which phenyl moiety may be substituted by $C_{1-6}$ alkyl, $C_{1-6}$ alkoxy, $CF_3$ or halogen, and $R_5$ is hydrogen; or $R_4$ and $R_5$ are attached to two adjacent carbon atoms and form together with these two carbon atoms a fused benzene ring, which benzene ring may be substituted by $C_{1-6}$ alkyl, $C_{1-6}$ alkoxy, $CF_3$ or halogen; as dopamine antagonists having use in the treatment of disorders of the gastro-intestinal function and/or in the treatment of emesis.

A novel class of compounds has now been discovered which is structurally distinct from the known compounds of the formula (I) but also has useful pharmacological activity, such as dopamine antagonist activity.

Accordingly the present invention provides a compound of the formula (I):

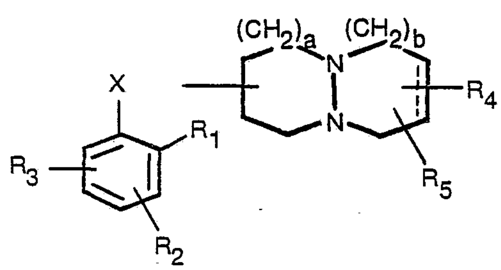

(I)

and pharmaceutically acceptable salts and N-oxides thereof, wherein:

a and b are separately 0, 1 or 2;

X is —CO—NR— wherein R is hydrogen or $C_{1-4}$ alkyl, or —NH—CO—;

$R_1$ is a $C_{1-6}$ alkoxy group;

$R_2$ and $R_3$ are the same or different and are hydrogen, halogen, $CF_3$, $C_{1-7}$ acyl, $C_{1-7}$ acylamino, $C_{1-6}$ alkyl-S(O)$_n$ wherein n is 0, 1 or 2, nitro or amino, $C_{1-6}$ alkoxy, aminocarbonyl or aminosulphonyl optionally substituted by one or two $C_{1-6}$ alkyl groups;

or $R_1$ and $R_2$ taken together an adjacent carbon atoms are methylenedioxy or ethylenedioxy in which case $R_3$ is any one of the groups given for $R_2$ and $R_3$ above; and either

$R_4$ is hydrogen, $C_{1-6}$ alkyl or phenyl; and $R_5$ is hydrogen; in which case when a and b are 0 or 1 and $R_4$ and $R_5$ are both hydrogen, then the dotted line may represent a double bond between the two adjacent carbon atoms (in the $R_4$/$R_5$ substituted ring) opposing the two adjacent nitrogen atoms; or

$R_4$ and $R_5$ are attached to two adjacent carbon atoms and form together with these two carbon atoms a fused benzene ring, which benzene ring may be substituted by $C_{1-6}$ alkyl, $C_{1-6}$ alkoxy, $CF_3$ or halogen;

there being a minimum of two carbon atoms between the amide nitrogen and each of the ring nitrogens.

Suitable examples of a include 0 or 1.

Suitable examples of b include 0 or 1.

Often when a is 0, b will be 1, and when a is 1, b will be 0.

a and b may also be both 1.

Favourably, X is —CONR—.

Suitable examples of R include hydrogen, methyl, ethyl and *n* and *iso*-propyl. More suitably R is hydrogen or methyl, preferably hydrogen.

Suitable examples of the group $R_1$ include methoxy, ethoxy and *n*- and *iso*-propoxy. Preferably $R_1$ is a methoxy group.

Suitable examples of $R_2$ and $R_3$ include the following atoms and groups: hydrogen, chlorine, bromine, $CF_3$, methoxy, formyl, acetyl, propionyl, *n*- and *iso*-butyryl, formylamino, acetylamino, propionylamino, *n*- and *iso*-butyrylamino, methyl, ethyl and *n*- and *iso*-propylsulphonyl, -sulphinyl and -thio, nitro, amino, aminocarbonyl and aminosulphone and amino, aminocarbonyl and aminosulphone substituted by one or two methyl, ethyl, *n*- or *iso*-propyl groups.

It is generally preferred that $R_2$ is in the 4-position relative to the carbonyl side chain, for greater activity in the resultant compound of the formula (I). For the same reason it is generally preferred that $R_3$ is in the 5-position relative to the carbonyl side chain.

In one particularly useful group of compounds, $R_2$ is 4-amino or 4-acylamino as defined, preferably 4-amino; and $R_3$ is 5-halo, preferably 5-chloro.

In a second particularly useful group of compounds, $R_2$ is hydrogen, 4-halo (eg chloro), 4-amino or 4-acylamino as defined, preferably hydrogen; and $R_3$ is 5-$C_{1-6}$ alkyl $S(O)_n$ (such as 5-methylsulphonyl, 5-methylsulphinyl or 5-methylthio), or 5-optionally alkylated aminosulphonyl.

Suitable examples of $R_4$ include hydrogen; methyl, ethyl, *n*- and *iso*-propyl, *n*-, *sec* and *tert*-butyl; and phenyl.

Preferred examples of $R_4$ include hydrogen; methyl; and phenyl. Preferably $R_4$ will substitute a carbon atom that is adjacent to a nitrogen atom in the ($R_4$/$R_5$ substituted) ring.

When $R_4$ and $R_5$ represent a fused benzene ring, suitable optional substituents of that fused benzene ring, include methyl, ethyl, *n*- and *iso*-propyl, *n*-, *sec*- and *tert*-butyl; methoxy, ethoxy and *n*- and *iso*-propoxy; $CF_3$, fluoro, chloro and bromo.

The pharmaceutically acceptable salts of the compounds of the formula (I) include acid addition salts with conventional acids such as hydrochloric, hydrobromic, phosphoric, sulphuric, citric, tartaric, lactic, and acetic acid and the like.

The pharmaceutically acceptable salts of the compound of the formula (I) also include quaternary ammonium salts. Examples of such salts include salts with compounds such as $R_6$—Y wherein $R_6$ is $C_{1-6}$ alkyl, phenyl-$C_{1-6}$ alkyl or $C_{5-7}$ cycloalkyl, and Y is an anion of an acid. Suitable examples of $R_6$ include methyl, ethyl and *n*- and *iso*-propyl; and benzyl and phenyl ethyl. Suitable examples of Y include the halides such as chloride, bromine and iodide.

The skilled man will realise that the compounds of the formula (I) are capable of existing in a number of stereoisomeric forms, and have chiral centres. The invention extends to each of these stereoisomeric forms, and to mixtures thereof (including racemates). The different steroisomeric forms may be separated one from the other by the usual methods, or any given isomer may be obtained by stereospecific or asymmetric synthesis. It will be appreciated that compounds of formula (I) containing an $R_4$ substituent are capable of existing in 'cis' and 'trans' forms with respect to the X linkage.

Similarly the skilled man will appreciate that the equatorial and axial amides of the formula (I) (ie ArX—<} and ArX⊦<} are interconvertable and exist in a dynamic equilibrium through the inversion of the two cyclic nitrogen atoms.

A group of compounds within formula (I) consists of those wherein:

X is —CONR— wherein R is as defined in formula (I);

$R_2$ and $R_3$ are the same or different and are hydrogen, halogen, $CF_3$, $C_{1-7}$ acyl, $C_{1-7}$ acylamino, $C_{1-6}$ alkyl-$S(O)_n$ wherein n is 0, 1 or 2, nitro or amino, $C_{1-6}$ alkoxy, aminocarbonyl or aminosulphonyl optionally substituted by one or two $C_{1-6}$ alkyl groups; and

$R_4$ and $R_5$ are as defined in formula (I) except that a and b are 1 when the dotted line in formula (I) represents a double bond.

From the aforesaid it will be appreciated that suitably the moiety of formula (II):

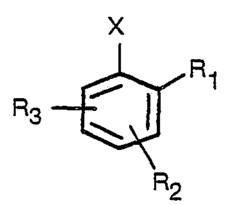

(II)

is a compound of formula (I) will have the structure (III):

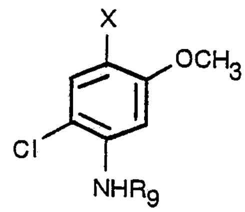

(III)

wherein $R_9$ is hydrogen or a $C_{1-4}$ alkanoyl group.

From the aforesaid it will be appreciated that one particularly useful sub-group of compounds within formula (I) is of formula (IV):

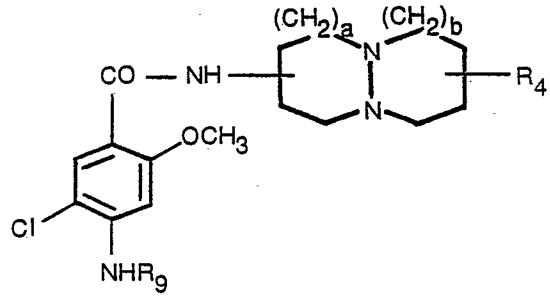

(IV)

wherein a, b and $R_4$ are as defined in formula (I) and $R_9$ is as defined in formula (III).

In formula (IV) the suitable and preferred examples of the variables are as described in relation to formula (I). Preferably a is 1 and b is 0.

A second particularly useful sub-group of compounds within formula (I) is of formula (V):

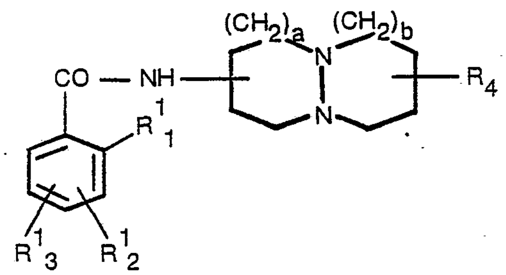

(V)

wherein a, b and $R_4$ are as defined in formula (I),

$R^1$ is $C_{1-6}$ alkoxy;

$R^1_1$ is $C_{1-6}$ alkoxy;

$R^1_2$ and $R^1_3$ are the same or different and are hydrogen, aminosulphonyl optionally substituted by one or two $C_{1-6}$ alkyl groups, $C_{1-6}$ alkylsulphonyl, $C_{1-6}$ alkylsulphinyl, $C_{1-6}$ alkoxy or hydroxy or

$R^1_1$ and $R^1_2$ taken together are methylenedioxy or ethylenedioxy, in which case $R^1_3$ is any one of the groups given above for $R^1_2$ and $R^1_2$.

Suitable and preferred examples of a, b and $R_4$ in formula (V) are as herein before described in relation to formula (IV).

$R^1_1$ is preferably methoxy.

Suitable examples of $R^1_2$ include hydrogen; chloro or bromo, amino, or acetyl amino. Preferably $R^1_2$ is hydrogen, chloro or amino.

Suitable examples of $R^1_3$ include methyl or ethylsulphonyl, -sulphinyl or -thio, and aminosulphonyl, and aminosulphonyl substituted by one or two methyl groups. Preferably $R^1_3$ is 5-methylsulphonyl, methylsulphinyl or aminosulphonyl (with respect to the 5-carbonyl side chain taken as 1) and $R^1_2$ is hydrogen.

In further useful sub-groups of compounds, the benzamido moiety is as shown in formula (IV) or (V), and the side chain is of formula (VI) or (VII):

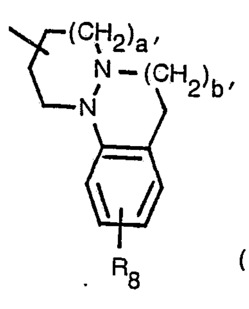

(VI)

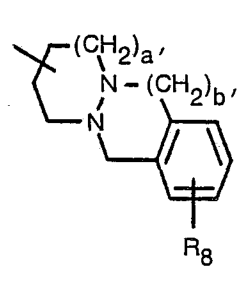

(VII)

wherein a' is 0 or 1, b' is 0 or 1 and $R_8$ is hydrogen, $C_{1-6}$ alkyl, $C_{1-6}$ alkoxy, $CF_3$ or halogen.

Suitable examples of $R_8$ include hydrogen and the groups listed hereinbefore as suitable optional substituents for the fused benzene ring that can be formed by $R_4$ and $R_5$ and the carbon atoms to which they are attached in formula (I).

Often a' is 0 and b' is 1 in formulae (VI) and (VII).

In yet further useful sub-groups of compounds, a is 0, b is 1 and $R_4$ is H in the compounds of formula (IV) or (V) and there is a double bond between the two adjacent carbon atoms (in the $R_4$ substituted ring) opposing the two adjacent nitrogen atoms.

Suitable and preferred examples of the variables in such compounds are as described in relation to formula (IV) and (V).

A further sub-group of compounds of interest are those of the formula (VIII):

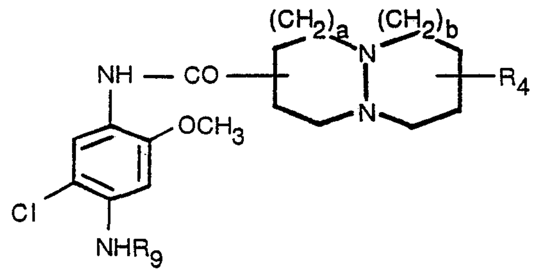

(VIII)

wherein the variables are as defined in formula (IV).

Suitable and preferred examples of the variables are as described in relation to formula (IV).

Another sub-group of compounds of interest are those of formula (IX):

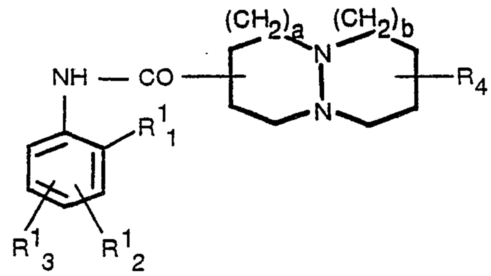

(IX)

wherein the variables are as defined in formula (V).

Suitable and preferred examples of the variables are as described in relation to formula (V).

In further sub-groups of compounds, the anilido moiety is as shown in formula (VIII) or (IX), and the side chain is of formula (VI) or (VII).

Suitable and preferred examples of the variables are as described in relation to formula (VI) and (VII).

The invention also provides a process for the preparation of a compound of the formula (I), which process comprises reacting a compound of the formula (X):

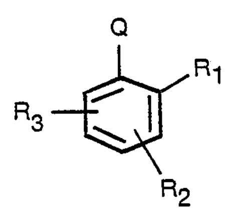

(X)

wherein Q is either COOH or a reactive derivative thereof or $H_2N$, with a compound of the formula (XI):

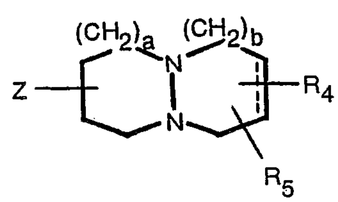

(XI)

wherein Z is HRN when Q is COOH or a reactive derivative thereof, or COOH or a reactive derivative thereof when Q is $H_2N$ and the remaining variables are as defined in formula (I); and thereafter if desired or necessary converting a group $R_2$ or $R_3$ in the thus formed compound to another group $R_2$ or $R_3$ respectively.

"Reactive derivative" when used herein means a derivative of the acid (X) or (XI) which can be reacted with the compound (XI) or (X) respectively to form an amido linkage between the acid group of the compound (X) or (XI) and the amino group of the compound of the formula (XI) or (X) respectively.

Often this reactive derivative will be the acid halide, such as the acid chloride, of the acid (X) or (XI). In such cases the reaction will normally be carried out in an inert solvent, preferably in the presence of an acid acceptor. The inert solvent can be any solvent inert to both reactants, such as benzene, toluene, diethyl ether or the like. The acid acceptor is suitably an organic base such as tertiary amine, e.g. triethylamine, trimethylamine, pyridine or picoline, or an inorganic acid acceptor, such as calcium carbonate, sodium carbonate, potassium carbonate or the like. It should be noted also that it is possible to use certain acid acceptors as the inert solvent, for example organic bases.

These reactions may be carried out at any non-extreme temperature such as −10°—100°C and more suitably 0°—80°C. The higher reaction temperatures are employed with less active acids of the formula (X) or (XI) whereas the lower temperatures are employed with the more reactive acids of the formula (X) or (XI).

Another useful reactive derivative of the acid (X) or (XI) that may be used in a highly activated ester, such as the pentachlorophenyl ester, when ambient temperatures may be used. The reaction is generally effected in an inert polar solvent, such as dimethylformamide.

The reaction may also be carried out by forming an anhydride of the acid (X) or (XI) in the usual manner, and reacting that with the compound (XI) and (X) respectively; normally a conventional mixed anhydride will be used.

Alternatively, the reactive derivative of the acid (X) or (XI) will be formed in situ, for instance by reacting the acid (X) or (XI) and the compound (XI) or (X) respectively in the presence of a dehydrating catalyst such as a carbodiimide, for example dicyclohexylcarbodiimide. The acid is preferably in the form of an acid addition salt, such as a hydrohalide, in particular the hydrochloride.

When Q is COOH or a reactive derivative thereof, the preferred process is wherein the acid is in the form of an acid halide as hereinbefore described.

When Q is $H_2N$ the preferred process is wherein the 'reactive derivative' is formed in situ using a dehydrating catalyst, as hereinbefore described.

The N-oxides of the compound of formula (I) may be prepared in conventional manner, for example by treatment with a per-acid, such as *m*-chloroperbenzoic acid.

The acid addition salts of the compounds of the formula (I) may be prepared in entirely conventional manner by reacting a compound of the formula (I) in base form with the chosen acid.

The quaternary ammonium salts of the compounds of the formula (I) may be prepared in conventional manner for such salts, such as by reaction of the chosen compound of the formula (I) with a compound $R_6Y$ as defined. This reaction is suitably carried out in an appropriate solvent such as acetone, methanol, ethanol, dimethylformamide and the like, at ambient or raised temperature and pressure.

The skilled man will appreciate that the choice or necessity of conversion of groups $R_2$ and/or $R_3$ to other groups $R_2$ and/or $R_3$ will be dictated by the nature and position of substituents $R_1$, $R_2$ and $R_3$.

Accordingly it will be realised that compounds of the formula (I) containing an $R_2$ or $R_3$ group which is convertible to another $R_2$ or $R_3$ group are useful intermediates, and as such form an important aspect of the invention.

By way of example of such conversions, the compounds of the formula (I) wherein $R_2$ or $R_3$ is a nitro group may be prepared via the nitration of the corresponding intermediate product wherein $R_2$ or $R_3$ is a hydrogen atom.

A particularly suitable nitrating agent for use in this process is fuming nitric acid in the presence of sulphuric acid. In general the reagent is added to a solution of the intermediate wherein $R_2$ or $R_3$ is hydrogen, in solution in an organic solvent such as acetic acid. Normally the reaction is carried out at or below ambient temperature, for example 0°—30°C and more suitably at about 5°—20°C subject to the reaction medium remaining fluid.

The nitro compound may be obtained from the reaction mixture by such conventional means as neutralisation followed by extraction into a water immiscible organic solvent such as ethyl acetate or dichloromethane from which it may be recovered by evaporation. If desired the nitro compound may be purified by chromatography or by recrystallisation of the free base or an acid addition salt thereof.

An optional process step provided by this invention in the preparation of the compounds of the formula (I) wherein $R_2$ or $R_3$ is an amino group comprises the reduction of a corresponding intermediate wherein $R_2$ or $R_3$ is a nitro group.

The reduction of the intermediates wherein $R_2$ or $R_3$ is a nitro group may be effected with reagents known to be suitable for reducing nitroanisole to aminoanisole. A suitable reagent for this reduction is stannous chloride in hydrochloric acid or in mixtures of hydrochloric and acetic acid. The desired amino compound may be obtained from the reaction mixture by respectively neutralisation followed by extraction into a water immiscible solvent such as ethyl acetate from which it may be recovered by evaporation of the solvent.

Another suitable method is catalytic hydrogenation at atmospheric pressure in polar solvent such as ethanol. Transition metal catalysts such as Raney nickel are often used. The desired compound may be obtained from the reaction mixture by filtration and evaporation to dryness.

The initial crude product in both cases may be purified by chromatography or crystallisation or by forming an acid addition salt which may be recrystallised.

In general however, when Q is COOH it is more convenient to prepare a compound of the formula (I) wherein $R_2$ or $R_3$ is an amino group from the corresponding $C_{1-7}$ acylamino acid or its reactive derivative, and to deacylate the compound of the formula (I) so formed.

Those compounds of the invention wherein $R_2$ or $R_3$ is a $C_{1-7}$ acylamino group may be prepared from the corresponding intermediate wherein $R_2$ or $R_3$ is an amino group by reaction with an acylating derivative, such as previously described as a suitable acylating derivative, e.g. of the acid of the formula (XIV). The reaction may proceed as described for the reaction of the compounds of the formula (XIV) and (XV). For an $R_2/R_3$ formamido group acylation may be effect with the free acid.

This invention thus also provides an optional process for the preparation of a compound of the formula (I) wherein $R_2$ or $R_3$ is an amino group which process comprises the deacylation of a corresponding intermediate wherein $R_2$ or $R_3$ is a $C_{1-7}$ acylamino group.

Generally the hydrolysis reaction may be effected by treatment with a base such as an alkali metal hydroxide.

Also a compound of the formula (I) wherein $R_2$ or $R_3$ is halogen may be prepared by a conventional halogenation of the corresponding intermediate wherein the said $R_2$ or $R_3$ is hydrogen.

Similarly the compounds wherein $R_2$ or $R_3$ is $C_{1-6}$ alkylthio or $C_{1-6}$ alkylsulphinyl may be oxidised to the corresponding compounds wherein $R_2$ or $R_3$ is $C_{1-6}$ alkylsulphinyl or $C_{1-6}$ alkylsulphonyl respectively.

These oxidations may conveniently be carried out conventionally at below ambient temperatures using an organic peracid in a non-aqueous inert rection medium preferably a chlorinated hydrocarbon solvent, for example using 3-chloroperbenzoic acid, or using a water soluble inorganic strong oxidant, such as an alkali metal permanganate or hydrogen peroxide in aqueous solution.

It will be appreciated by the skilled man that, depending on the other specific substituents in the compound of the formula (I), such as oxidation on a compound of the formula (I) may also form the N-oxide of the bicyclic moiety therein.

Given the specific substitution desired and having been decided whether the compound or its N-oxide is required, the skilled man will readily ascertain whether such $R_2/R_3$ interconversion is desirable. In general it is preferred to effect the oxidation in the intermediate of formula (X) before coupling.

The intermediates of formula (XI) wherein Z is HRN may be prepared by the reduction of an azide of formula (XII):

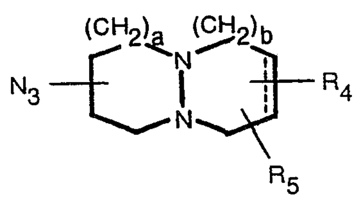

(XII)

This reduction is conveniently carried out with lithium aluminium hydride under the usual conditions.

This process of course yields a compound of the formula (XI) wherein R is hydrogen. If a compound of formula (XI) wherein R is alkyl is desired, it may simply be prepared from the corresponding R is hydrogen compound by mono-alkylation. This may be effected in any suitably manner, for example by acylation with an anhydride followed by reduction with lithium aluminium hydride.

The intermediates of formula (XI) wherein Z is COOH may be prepared by the hydrolysis of a compound of formula (XIII):

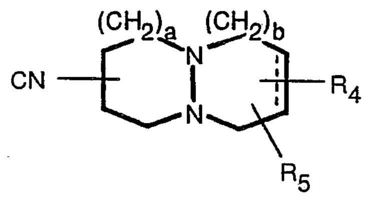

(XIII)

The hydrolysis is normally carried out under acid conditions.

Compounds of the formula (XII) may themselves be prepared by converting the hydroxy group in a compound of formula (XIV):

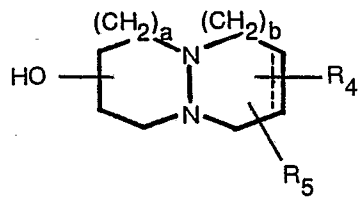

(XIV)

to azide.

This reaction may suitably be carried out in the general manner described in A K Bose et al, Tetrahedron Letters, 1977, *23*, 1977). For example the reaction may be effected with triphenyl phosphine, diethyl azadicarboxylate and diphenyl phosphonyl azide.

Compounds of formula (XIII) may be prepared by converting the hydroxy group in a compound of formula (XIV) into nitrile by standard methods, for example by converting the hydroxy group firstly to a good leaving group such as a benzene sulphonyl ester group and then treating with sodium cyanide.

Compounds of the formula (XIV) may themselves be prepared by the reductive hydroboration and oxidation of a compound of formula (XV):

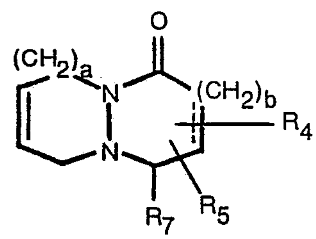

(XV)

wherein $R_7$ is a carbonyl group, or a $R_4$ $C_{1-4}$ alkyl or phenyl group.

When the optional double bond is present in the compound of formula (XV) then naturally the quantities of reactants used in the hydroboration are carefully controlled.

The reaction is suitably carried out by treatment with a borane·tetrahydrofuran complex in tetrahydrofuran with subsequent oxidation with alkaline peroxide under the usual conditions.

Alternatively, compounds of the formula (XIV) wherein a is 0 may be prepared by reacting a compound of formula (XVI):

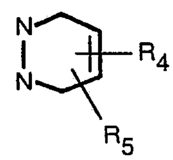

(XVI)

with epichlorohydrin under basic conditions in an inert solvent such as with potassium carbonate in ethanol.

Compounds of the formula (XV), (XVI) and also compounds of the formula (X), are either known compounds or may be prepared in analogous manner to known compounds.

As hereinbefore stated, the compounds of the formula (I) are dopamine antagonists.

Depending on their balance between peripheral and central action, the compounds of the formula (I) may be used in the treatment of disorders related to impaired gastro-intestinal motility, such as retarded gastric emptying, dyspepsia, flatulence, oesophagal reflux, peptic ulcer and emesis, and/or in the treatment of disorders of the central nervous system, such as psychosis.

Compounds of particular interest for their anti-emetic activity and for their beneficial effect on gastric motility are those wherein X is —CO—NR—. Compounds which are of interest for their CNS activity are those wherein X is —NH—CO—. Examples of compounds of interest for their beneficial effect on gastric motility are the quaternary ammonium salts and N-oxides of the compounds of formula (I).

All the compounds of the formula (I) may be used in the treatment of emesis.

The invention therefore also provides a pharmaceutical composition comprising a compound of the formula (I), or a hydrate or a pharmaceutically acceptable salt thereof, together with a pharmaceutically acceptable carrier. Such compositions may be adapted for oral or parenteral administration, and as such may be in the form of tablets, capsules, oral liquid preparations, powders, granules, lozenges, reconstitutable powders, injectable and infusable solutions or suspensions; the compositions may also be in the form of suppositories. Normally, orally administrable compositions are preferred.

Tablets and capsules for oral administration may be in unit dose presentation form, and may contain conventional excipients such as binding agents, fillers, tabletting lubricants, disintegrants, and acceptable

wetting agents. The tablets may be coated according to methods well known in normal pharmaceutical practice. Oral liquid preparations may be in the form of, for example, aqueous or oily suspensions, solutions, emulsions, syrups, or elixirs, or may be presented in a dry product for reconstitution with water or other suitable vehicle before use. Such liquid preparations may contain conventional additives such as suspending agents, emulsifying agents, non-aqueous vehicles (which may include edible oils), preservatives, and if desired conventional flavouring or colouring agents.

For parenteral administration, fluid unit dosage forms are prepared utilizing the compound of the formula (I) and a sterile vehicle. The compound, depending on the vehicle and concentration used, can be either suspended or dissolved in the vehicle. In preparing solutions the compound can be dissolved for injection and filter sterilized before filling into a suitable vial or ampoule and sealing. Advantageously, adjuvants such as a local anesthetic, preservatives and buffering agents can be dissolved in the vehicle. Parenteral suspensions are prepared in substantially the same manner except that the compound is suspended in the vehicle instead of being dissolved and sterilized by exposure to ethylene oxide before suspending in the sterile vehicle. Advantageously, a surfactant or wetting agent is included in the composition to facilitate uniform distribution of the compound.

As is common practice, the compositions will usually be accompanied by written or printed directions for use in the medical treatment concerned.

It will of course be realised that the precise dosage used in the treatment of any of the hereinbefore described disorders will depend on the actual compound of the formula (I) used, and also on other factors such as the seriousness of the disorder being treated.

The compounds of the present invention are suitable for the treatment of maladies in mammals including humans comprising the administration of an effective amount of a compound of the formula (I) or a pharmaceutically acceptable salt thereof. The "effective amount" will depend in the usual way on a number of factors such as the nature and severity of the malady to be treated, the weight of the sufferer, and the actual compound used.

However by way of illustration, unit doses will suitably contain 0.1 to 20 mgs of the compound of formula (I), for example 0.5 to 10 mgs.

Again by way of illustration, such unit doses will suitably be administered more than once a day, for example 2, 3, 4, 5 or 6 times a day, in such a way that the total daily dose is suitably in the range 0.01 to 10 mg/kg per day.

Compounds of the formula (I) have the ability to potentiate the effect of conventional analgesics in migraine treatment when administered concurrently with the analgesic.

Thus the invention provides a pharmaceutical composition comprising a compound of the formula (I) and an analgesic.

The compound of the formula (I) and the analgesic , such as 2-(acetyloxy) benzoic acid or paracetamol, will be present in the composition in amounts generally similar to their usual effective dose.

The composition can be a combination product, for example a tablet or capsule containing both a compound of the formula (I) and an analgesic for oral administration, or a twin pack comprising the two active ingredients made up for separate administration.

The compounds of the present invention accordingly are suitable for the treatment of migraine comprising the administration to the sufferer of a compound of the formula (I) and an analgesic.

The following Examples illustrate the preparation of compounds of formula (I).

The following Descriptions illustrate the preparation of intermediates.

All temperatures are in degrees centrigrade.

*Description 1* (Intermediate for Examples 1 and 2)

(a) *(±) 3-Hydroxy-1,6-diazabicyclo [4,4,0] decane (D.1a)*

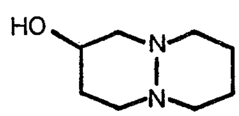

(D.1a)

To a stirred solution of 1,6-diazabicyclo [4,4,0]-8-decene-2,5-dione (4.2 g) in dry THF (100 mls) at 0°C was added a 1M solution of borane — THF complex in THF (100 mls) and the reaction mixture was refluxed for 2 hours. On cooling to 0°C, 2.5N aqueous sodium hydroxide solution (10 mls) and 12% aqueous hydrogen peroxide (20 mls) were added and the mixture stirred at room temperature for 1 hour. An excess of dilute hydrochloric acid (5N, 60 mls) was then added and the reaction mixture stirred overnight. Removal of the solvent in vacuo, basification with saturated potassium carbonate and extraction with methylene chloride gave, on column chromatography (Alumina + 5% water, ethyl acetate) the (±) 3-hydroxy-1,6-diazabicyclo [4,4,0] decane (D.1a) (2.6 g, 50%).

m.p. 95—7°

i.r. (KBr disc) $\nu_{(OH)}$ 3460, 3350, 3190 $m^{-1}$

m.s. M$^{\oplus}$ 156, 1257 (Theory 156.1252).

(b) *(±) 3-Azido-1,6-diazabicyclo [4.4.0] decane (D.1b).*

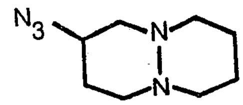

(D.1b)

To a stirred solution of triphenyl phosphine (4.2 g) in dry THF (100 ml) at 0°C were added, sequentially at 10 minute intervals, diethyl azidocarboxylate (2.7 g), (±) 3-hydroxy-1,6-diazabicyclo [4,4,0]-decane (D.1a) (2.34 g) and diphenyl phosphoryl azide (4.4 g) and the whole stirred at room temperature for 3 days. The solvent was removed in vacuo and the residue treated with ethyl acetate. Extraction with 5N hydrochloric acid, basification with potassium carbonate and extraction with methylene chloride gave the crude (±) 3-azido-1,6-diazabicyclo [4,4,0] decane (D.1b) (2.2 g, 80%).

i.r. (film) $v_{(N_3)}$ 2090 $m^{-1}$

(c) *(±) 3-Amino-1,6-diazabicyclo [4,4,0] decane (D.1c)*

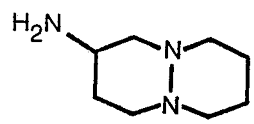

(D.1c)

The crude (±) 3-azido-1,6-diazabicyclo [4,4,0] decane (D.1b) (2.2 g) was reduced with lithium aluminium hydride (0.6 g) in ether (100 mls) for 4 hours. Hydrolysis, extraction with methylene chloride and solvent removal gave the crude (±) 3-amino-1,6-diazabicyclo [4,4,0] decane (D.1c) (1.6 g, 85%).

Description 2

(a) *(±) 3-Hydroxy-1,5-diazabicyclo[4,3,0]nona-7-ene (D.2a)* intermediate for Examples 3 and 4

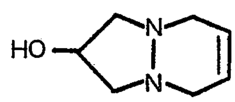

(D.2a)

A solution of 1,2,3,6-tetrahydropyridazine (3 g), epichlorohydrin (3.3 g) and potassium carbonate (5 g) in ethanol (100 ml) was heated under reflux for 2 hours. On cooling, the reaction mixture was concentrated in vacuo and the residue extracted with methylene chloride. Trituration of the residue after removing the methylene chloride afforded the (±) 3-hydroxy-1,5-diazabicyclo[4,3,0]nona-7-ene (D.2a) (3.3 g, 65%).

m.p. 82—5°

m.s. $M^+$ 140.0957 (Theory 140.0965).

Following the procedures outlined in Description 2(a), the following compounds were prepared:

(2b) *(±) 3-Hydroxy-1,5-diazabicyclo[4,3,0]nonane (D.2b)* (43%) intermediate for Examples 5 and 6

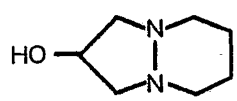

(D.2b)

m.s. $M^+$ 142.1108 (Theory 142.1110).

(2c) *(±) 3-Hydroxy-7-phenyl-1,5-diazabicyclo[4,3,0]nonane (D.2c)* (50%) intermediate for Examples 7 and 8

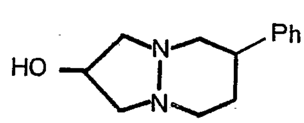

m.s. $M^+$ 218.1409 (Theory 218.1399).

(2d) *(±) 2-Hydroxy-1H-2,3,5,10-tetrahydro-pyrozala[1,2-b]pthalazine (D.2d)* (54%) intermediate for compounds 9 and 10

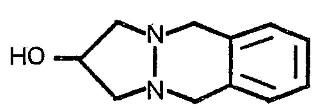

m.p. 122—5°

m.s. $M^+$ 190.1133 (Theory 190.1160).

Description 3

*(±) 7-Hydroxy- and (±) 8-Hydroxy-2-methyl-1,5-diazabicyclo[4,3,0]nonan-4-one (D.3)* intermediate for compounds 11, 12, 13, 14, 15 and 16

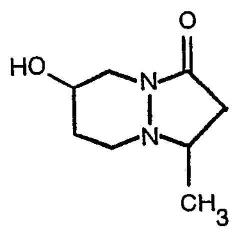
+
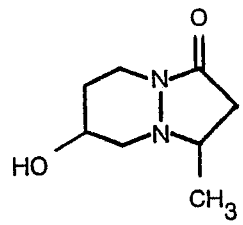
(D.3)

A solution of (±) 4-hydroxy-1,2-dicarbethoxy-hexahydropyridazine (26 g) and potassium hydroxide (25 g) in 400 ml of ethanol was heated under reflux for 24 hours. On cooling, acetic acid (20 ml) and methyl crotonate (15 ml) were added and the mixture re-heated to reflux for 12 hours. On concentration in vacuo, saturated potassium carbonate solution was added and extracted with methylene chloride. Removal of the solvent gave the isomeric mixture of (±) 7-hydroxy and (±) 8-hydroxy-2-methyl-1,5-diazabicyclo[4,3,0]nonan-4-ones (D.3) (9.1 g, 68%).

b.p. 120—40°/0.1 mm

m.s. $M^+$ 170.1059 (Theory 170.1064).

Description 4

*(±) 7-Hydroxy- and (±) 8-Hydroxy-2-methyl-1,5-diazabicyclo[4,3,0]nonane (D.4), 12, 13, 14, 15 and 16*

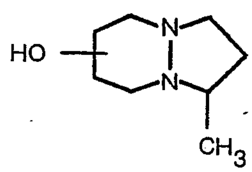
(D.4)

To a stirred suspension of lithium aluminium hydride (2 g) in dry THF (200 mls) was added the isomer mixture of (±) 7-hydroxy- and (±) 8-hydroxy-2-methyl-1,5-diazabicyclo[4,30]nonane-4-one (D.3) (9.1 g) and the whole heated under reflux for 12 hours. Standard work-up afforded the isomer mixture of (±) 7-hydroxy and (±) 7-hydroxy and (±) 8-hydroxy-2-methyl-1,5-diazabicyclo[4,3,0]nonanes (D.4) (5.5 g, 66%).

Description 5

Following the procedures outlined in Description 1(b), the following compounds were prepared:

(a) *(±) 3-Azido-1,5-diazabicyclo[4,3,0]nona-7-ene (D.5a)* (ca. 100%) intermediate for Examples 3 and 4

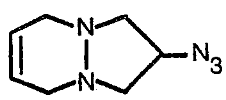
(D.5a)

(b) *(±) 3-Azido-1,5-diazabicyclo[4,3,0]nonane (D.5b)* (ca. 100%) intermediate for Examples 5 and 6

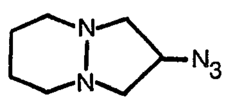
(D.5b)

(c) *(±) 3-Azido-7-phenyl-1,5-diazabicyclo[4,3,0]nonane (D.5c)* (80%) intermediate for Examples 7 and 8

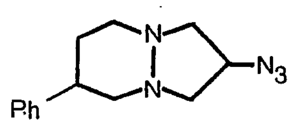
(D.5c)

m.s. $M^+$ 243.1475 (Theory 243.1466)

(d) *(±) 2-Azido-1H-2,3,5,10-tetrahydro-pyrazo[1,2-b]phthalazine (D.5d)* (58%) intermediate for Examples 9 and 10

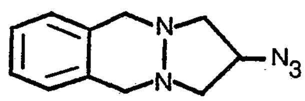

(e) *(±) 7-Azido- and (±) 8-Azido-2-methyl-1,5-diazabicyclo[4,3,0]nonane (D.5d)* (37%) intermediates for Examples 11, 12, 13, 14, 15 and 16

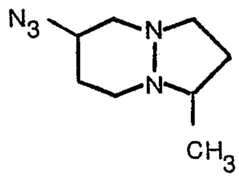

+

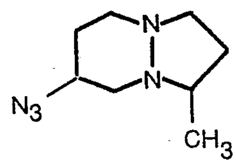

(D.5e)

Description 6

Following the procedures outlined in Description 1(c), the following compounds were prepared:

(a) *(±) 3-Amino-1,5-diazabicyclo[4,3,0]nonan-7-ene (D.6a)* (ca. 100%) intermediate for Examples 3 and 4

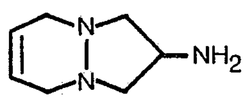

(D.6a)

(b) *(±) 3-Amino-1,5-diazabicyclo[4,3,0]nonane (D.6b)* (50%) intermediate for Examples 5 and 6

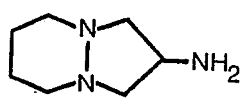

(D.6b)

b.p. 58—60°/1 mm

(c) *(±) 3-Amino-7-phenyl-1,5-diazabicyclo[4,3,0]nonane (D.6c)* (95%) intermediate for Examples 7 and 8

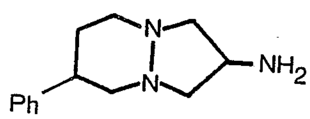

(D.6c)

(d) *(±) 2-Amino-1H-2,3,5,10-tetrahydro-pyrazol[1,2-b]phthalazine (D.6d)* (85%) intermediate for Examples 9 and 10

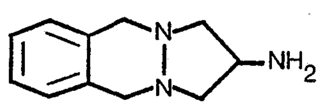

(D.6d)

m.s. $M^+$ 189.1275 (Theory 189.1286)

(e) *(±) 7-Amino- and (±) 8-Amino-2-methyl-1,5-diazabicyclo[4,3,0]nonane (D.6c)* (100%) intermediates for Examples 11, 12, 13, 14, 15 and 16

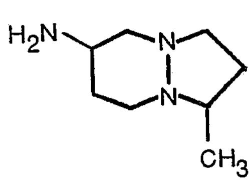

+

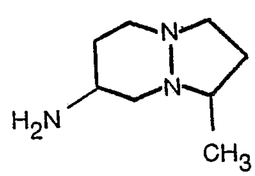

(D.6e)

Example 1

*(±) 4-Acetamido-5-chloro-2-methoxy-N-(3-{1,6-diazabicyclo[4,4,0]decyl}) benzamide* (1)

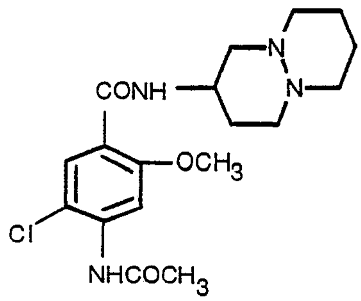

(1)

To 4-acetamido-5-chloro-2-methoxybenzoyl chloride (2.9 g) in toluene (150 mls) and triethylamine (4 ml) was added the crude (±) 3-amino-1,6-diazabicyclo[4,4,0]decane (D.1c) (1.6 g) in toluene (20 ml). The reaction mixture was stirred at room temperature for 4 hours, then treated with 2.5N aqueous sodium hydroxide (10 ml. The toluene layer was separated, the aqueous layer extracted with chloroform (3 × 100 mls), and the combined organic extracts dried ($K_2CO_3$). The solvent was removed and the residue chromatographed (Alumina + 5% water, chloroform) to give the (±) 4-acetamido-5-chloro-2-methoxy-N-(3-{1,6-diazabicyclo[4,4,0]decyl}) benzamide )1) as an oil (2.8 g, 70%).

n.m.r. (δ, $CDCl_3$)

9.0—7.5 (m, 2H, CONH̲ CH<, $CH_3$, CONH̲—Ar).

8.34 (S, 1H, aryl-H̲).

8.23 (S, 1H, aryl-H̲)

4.5—3.8 (m, 4H, CONH CH̲< including 4.03, broad S, 3H, —$OCH_3$).

3.4—1.0 (m, 17H, remaining H̲ including 2.28, S, 3H, —COCH̲₃)

Example 2

*(±) 4-Amino-5-chloro-2-methoxy-N-(3-{1,6-diazabicyclo[4,4,0]decyl}) benzamide* (2)

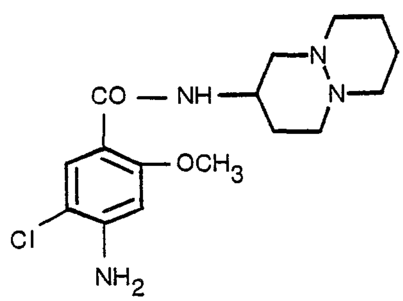

(2)

(1) (2.8 g) (prepared as in Example 1) was refluxed with an aqueous ethanolic sodium hydroxide solution (2.5N, 6 ml; 30 ml of ethanol) for 2 hours. The mixture was then cooled to room temperature and the ethanol removed in vacuo. The residue was extracted with chloroform (2 × 50 ml) and dried ($K_2CO_3$). Filtration and removal of solvent gave the crude product, which wash recrystallised from ethyl acetate to give (±) 4-amino-5-chloro-2-methoxy-N-(3-{1,6-diazabicyclo[4,4,0]decyl}) benzamide (2) (1.6 g, 65%).

m.p. 154—5°.

n.m.r. (δ, $CDCl_3$)

9.6—8.4, 7.7—7.3 (m, 1M, —CONH̲ CH<)

8.14 (S, 1H, aryl 6-H̲)

6.31 (S, 1H, aryl 3-H̲)

4.5—3.7 (m, 6H, aryl-NH̲₂, CONH—CH̲< and 3.95, broad s, 3H, —OCH̲₃).

3.3—1.0 (m, 14H, remaining protons).

Following the procedures outlined in Examples 1 and 2, the following 4-amino and 4-acetamido compounds were prepared:

Example 3

*(±) 4-Acetamido-5-chloro-2-methoxy-N-(3-{1,5-diazabicyclo[4,3,0]nona-7-enyl}benzamide* (3) (74%)

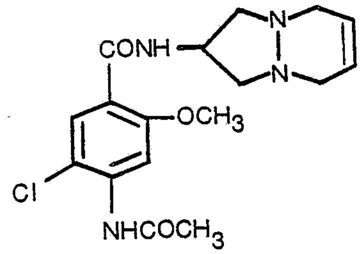

(3)

m.p. 164—5°
m.s. $M^+$ 364.1306 (Theory 364.1210)
n.m.r. (δ, $CDCl_3$)
8.3—7.4 (m, 2H, CONHCH<, $CH_3CONHAr$)
8.2 (s, 1H, Aryl H)
7.97 (s, 1H, Aryl H)
5.7—5.5 (br.s. 2H, =CH—)
5.0—4.4 (m, 1H, NHCH<)
4.0—2.0 (m, 14H, remaining H including 3.87, s, 3H, $OCH_3$ and 2.20, s, 3H, $COCH_3$)

Example 4

*(±0) 4-Amino-5-chloro-2-methoxy-N-(3-{1,5-diazabicyclo[4,3,0]nona-7-enyl}) benzamide* (4) (67%)

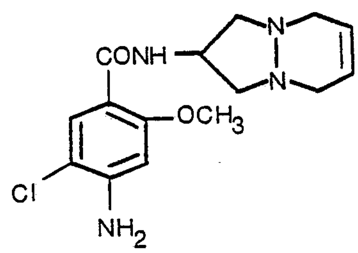

(4)

m.p. 196—8°
m.s. $M^+$ 322.1199 (Theory 322. 1201)
n.m.r. (δ, $CDCl_3$)
8.06 (s, 1H, Aryl 6H)
8.1—7.8 (m, 1H, CONH)
6.27 (s, 1H, Aryl 3H)
5.85—5.75 (br.s. 2H, =CH—)
5.05—4.65 (m, 1H NHCH<).
4.6—4.35 (m, 2H, $—NH_2$)
3.85 (s, 3H, $OCH_3$)
3.5—3.1 (m, 6H, $NCH_2CH=$ and >CH—N).
2.85—2.6 (m, 2H, >CH—N).

Example 5

*(±) 4-Acetamido-5-chloro-2-methoxy-N-(3-{1,5-diazabicyclo[4,3,0]nonyl})benzamide* (5) (73%)

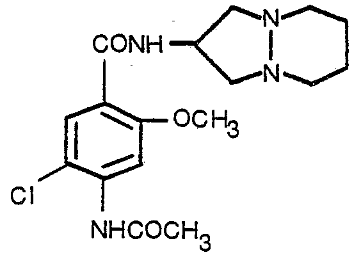

(5)

m.p. 165—9°
m.s. $M^+$ 366.1462 (Theory 366.1466).

Example 6

*(±) 4-Amino-5-chloro-2-methoxy-N-(3-{1,5-diazabicyclo[4,3,0]nonyl})benzamide* (6) (70%)

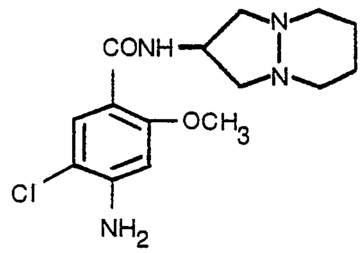

(6)

m.p. 195—8°
m.s. $M^+$ 324.1364 (Theory 324.1375)
n.m.r. (δ, $CDCl_3$)
8.06 (s, 1H, Aryl 6H)
8.0—7.7 (m, 1H, CONH).
6.27 (s, 1H, Aryl 3H)
48—4.3 (m, 3H, $NH_2$ and NHCH<)
3.85 (s, 3H, $OCH_3$).
3.5—1.4 (m, 12H, remaining protons).

Example 7

*(±) 4-Acetamido-5-chloro-2-methoxy-N-(3-{7-phenyl-1,5-diazabicyclo[4,3,0]nonyl})benzamide* (7) (70%)

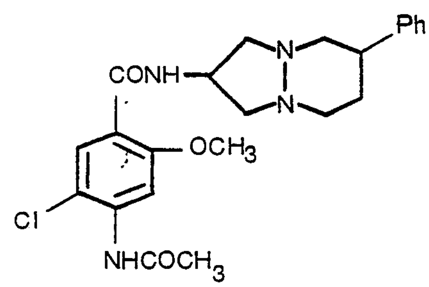

(7)

m.s. $M^+$ 442.1796 (Theory 442.1823)
n.m.r. (δ, $CDCl_3$)
8.12 (s, 1H, Aryl H)
8.05 (s, 1H, Aryl H)
8.0—7.5 (m, 2H, CONH—, $CH_3CONH$)
7.3—7.0 (br.s. 5H, Aryl H)
4.5—3.8 (m, 4H, CHNH— including 3.81, s, —$OCH_3$)
3.5—0.9 (m, 14H, remaining protons including 2.19, s, 3H, $COCH_3$)

Example 8

*(±) 4-Amino-5-chloro-2-methoxy-N-(3-{7-phenyl-1,5-diazabicyclo[4,3,0]nonyl}) benzamide* (8) (64%)

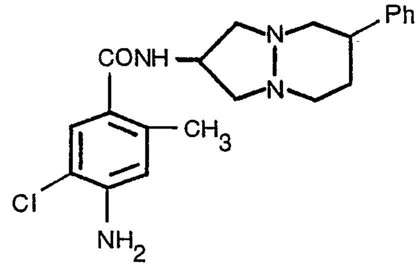

(8)

m.p. 147—8°
m.s. $M^+$ 400.1666 (Theory 400.1668)
n.m.r. (δ, $CDCl_3$)

8.07 (s, 1H, Aryl 6H)
8.05—7.85 (m, 1H, CONH).
7.4—7.1 (m, 5H, Aryl H)
6.27 (s, 1H, Aryl 3H)
5.0—4.25 (m, 3H, NHCH< and $NH_2$)
4.0—3.6 (m, 4H, ArCH< including 3.87, s, 3H, $OCH_3$)
3.4—1.8 (m, 10H remaining protons)

Example 9

*(±) 4-Acetamido-5-chloro-2-methoxy-N-(2-{1H-2,3,5,10-tetrahydropyrazolo[1,2-b]phthalazinyl})benzamide* (9) (75%)

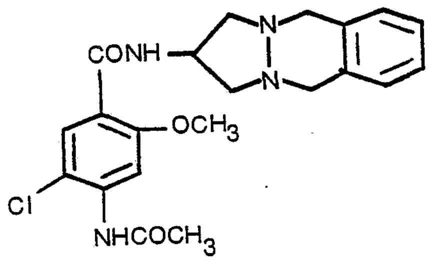

(9)

m.p. 210—4°
m.s. $M^+$ 414.1472 (Theory 414.1486)
n.m.r. (δ, $CDCl_3$)
8.27 (s, 1H, Aryl H)
8.17 (s, 1H, Aryl H)
8.25—7.7 (m, 2H, CONH, $CH_3$CONH)
7.25—6.95 (m, 4H, Aryl H)
5.1—4.7 (m, 1H, NHCH<).
3.93 (s, 4H, Aryl $CH_2$N)
3.89 (s, 3H, $OCH_3$).
3.5—3.2 (m, 2H, >CH—N)
3.0—2.7 (m, 2H, >CH—N).
2.25 (s, 3H, $COCH_3$).

Example 10

*(±) 4-Amino-5-chloro-2-methoxy-N-(2-{1H-2,3,5,10-tetrahydropyrazalo[1,2-b]phthalazinyl})benzamide* (10) (65%)

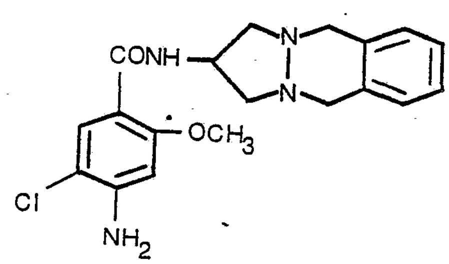

(10)

m.p. 229—31°.
n.m.r. (δ, $CDCl_3$ + $d^6$DMSO).
8.1—7.9 (brd, 1H, CONH).
7.79 (s, 1H, Aryl, 6H)
7.25—6.95 (brs, 4H, Aryl H).
6.50 (s, 1H, Aryl 3H)
5.75—5.6 (brs, 2H, $NH_2$)
4.8—4.45 (m, 1H, NHCH<).
3.86 (s, 4H, N—$CH_2$—Aryl).
3.82 (s, 3H, $OCH_3$).
3.45—3.1 (m, 2H, N—CH<).
2.9—2.6 (m, 2H, N—CH<).

Examples 11, 12 and 13

Three Isomers I, II and III of *(±) 4 - Acetamido - 5 - chloro - 2 - methoxy - N - (7 - {2 - methyl - 1,5 - diazabicyclo[4,3,0]nonyl})benzamide and (±) 4 - Acetamido - 5 - chloro - 2 - methoxy - N - (8 - {2 - methyl - 1,5 - diazabicyclo[4,3,0]nonyl{})benzamide* (11) (12) and (13) (total 94%).

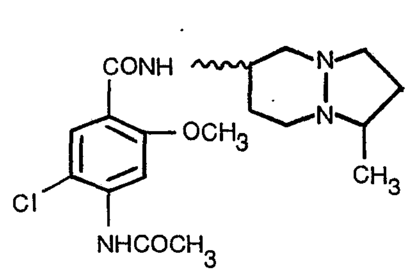

and

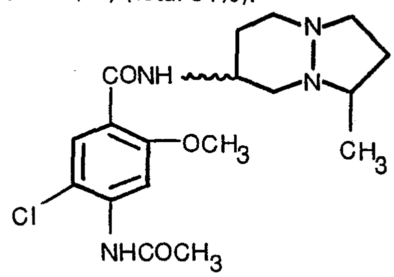

(4 possible isomers)

From a column chromatography (silica: ethyl acetate/10% methanol), three structural/geometric isomers were isolated.

Isomer I (II) (18%)

n.m.r. (δ, $CDCl_3$).

8.85—8.45 (brd, 1H, CONH)
8.16 (s, 1H, Aryl H).
8.08 (s, 1H, Aryl H)
8.2—7.9 (m, 1H, CONH).
4.5—3.8 (m, 4H, NHCH< including 3.92, s, 3H, $OCH_3$).
3.4—1.0 (m, 17H, remaining H including 2.25, s, 3H, $COCH_3$ and 1.13, d, 3H, $CH_3CH<$ J=6Hz).

Isomer II (12) (36%)

n.m.r. (δ, $CDCl_3$)

8.18 (s, 1H, Aryl H).
8.10 (s, 1H, Aryl H).
8.2—7.5 (m, 2H, CONH).
4.6—1.0 (m, 21H, remaining protons including 3.87, s, 3H, $OCH_3$; 2.24, s, 3H, $COCH_3$, 1.12, d, 3H, $>CHCH_3$, J=6$H_z$)

Isomer III (13) (40%)

m.s. $M^+$ 380.1646 (Theory 380.1677).

n.m.r. (δ, $CDCl_3$)

8.15 (s, 1H, Aryl H)
8.07 (s, 1H, Aryl H).
8.2—8.0 (m, 1H, CONH)
7.8—7.5 (brd, 1H, CONH).
4.4—1.0 (m, 21H, remaining protons including 3.89, s, 3H, $OCH_3$; 2.27, s, 3H, $COCH_3$; 1.12, d, 3H $>CHCH_3$ J=5$H_z$).

*Note* All CONH n.m.r. (δ) values are concentration dependent.

Example 14

*Isomer I of (±) 4-Amino-5-chloro-2-methoxy-N-(7 or 8-{2-methyl-1,5-diazabicyclo[4,3,0]nonyl})benzamide* (14) (62%).

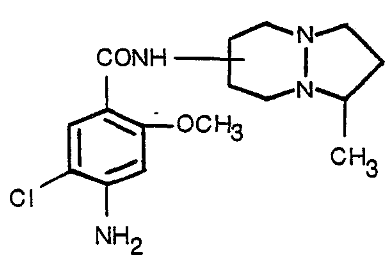

Isomer I (14)

m.p. 203—5°

m.s. $M^+$ 338.1487 (Theory 338.1465).

n.m.r. (δ$CDCL_3$)

8.75—8,40 (brd, 1H, CONH).
8.07 (s, 1H, Aryl 6H).
6.29 (s. 1H, Aryl 3H).
4.6—4.25 (m, 3H, $NH_2$ and NHCH<).
3.87 (s, 3H, $OCH_3$).
3.25—1.05 (m, 14H, remaining protons including 1.15, d, 3H, CH, $CH_3$, J=6$H_z$).
*Note* CONH n.m.r. (δ) values are concentration dependent.

## Example 15

*Isomer II of (±) 4-Amino-5-chloro-2-methoxy-N-(7- or 8-{2-methyl-1,5-diazabicyclo[4,3,0]nonyl})benzamide* (15) (28%).

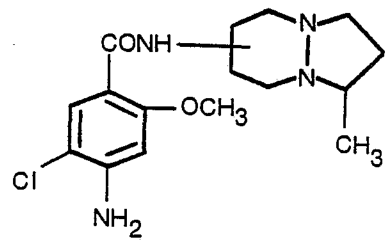

Isomer II (15)

m.p. 164—6°.
m.s. $M^+$ 338.1516 (Theory 338.1525).
n.m.r. (δ $CDCl_3$)
8.08 (s, 1H, Aryl 6H).
7.75—7.50 (brd, 1H, CONH).
6.29 (s, 1H, Aryl 3H).
4.7—1.0 (m, 20H, remaining protons including 3.83, s, 3H, $OCH_3$; 1.15, d, 3H, $CHCH_3$, J=6$H_z$).
*Note* CONH n.m.r. (δ) values are concentration dependent.

## Example 16

*Isomer III of (±) 4-Amino-5-chloro-2-methoxy-N-(7 or 8-{2-methyl-1,5-diazabicyclo[4,3,0]nonyl})benzamide* (16) (70%)

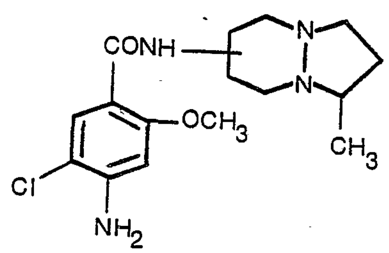

Isomer III (16)

m.p. 187—9°.
m.s. $M^+$ 338.1520 (Theory 338.1531)
n.m.r. (δ $CDCl_3$)
8.10 (s, 1H, aryl H)
7.65—7.4 (brd, 1H, CONH)
6.28 (s, 1H, aryl H)
4.5—3.7 (m, 6H $NH_2$, NHCH< including 3.86, s, 3H, $OCH_3$).
3.6—1.0 (m, 14H, remaining protons including 1.13, d, 3H, $CHCh_3$, J=6Hz).
*Note* CONH n.m.r. (δ) values are concentration dependent.

Biological Data Section
Gastric Activity
Increase in intragastric pressure in the rat.

Intragastric pressure changes were recorded from previously starved conscious but retained rats using a saline filled catheter inserted into the lumen of the stomach *via* a permanent gastric fistula. The catheter was connected to a physiological pressure transducer and pressure changes recorded on a hot wire pen recorder. In each animal a pre-dose period of 40 minutes was allowed to obtain a measure of spontaneous activity. An index of activity was obtained by measuring the average height of pressure waves during 10 minute periods. Values for 4 such periods were obtained during assessment of spontaneous activity and for the 40 minute period after the subcutaneous administration of the compounds. Students 't' test was applied to the difference in average values obtained for spontaneous and post-compound activity.

Table I shows the minimum dose for activity. A significant effect was taken as occurring if a p value of less than 0.05 (2-tailed) was obtained.

TABLE I

| Compound of Example No. | Dose mg/kg Subcutaneously |
|---|---|
| 2 | 1.0 |
| 6 | 0.5 |
| 14 | 0.5 |
| 15 | 1.0 |

Inhibition of apomorphine induced climbing in the mouse

The test is based on that described by Protais, P., Constantin, J. and Schwartz, J.C. (1976), Psychopharmacology, *50*, 1—6.

Apomorphine 1 mg/kg s.c. induces mice to climb the wall of a wire cage (inverted food hopper — 11 × 7.5 × 18 cm high). Mice acclimatised in their home cages in groups of 5 are placed under the hoppers immediately after the injection of apomorphine 1 mg/kg s.c. At 10, 20 and 30 minutes after injection climbing behaviour is scored. The mice are observed for 30 seconds and scored according to the position in which they spend the majority of time, score 0 — four paws on floor of cage; score 1 — fore paws only on walls; score 2 — all paws on wall of cage. The scores at all 3 times and for each mouse are summed and mice drug treated subcutaneously compared to mice receiving apomorphine only. A saline only treated groups is also included and any score, generally <5% of maximum taken into account.

Compounds were also tested for their ability to inhibit apomorphine induced climbing in the mouse. Inactivity in this test may be indicative of a low propensity to produce extra-pyramidal side-effects in man.

$ED_{50}$ values were obtained according to the method of Litchfield and Wilcox in J. Pharmacol. 1949, *96*, 99—113.

The results were as set out in Table 2.

TABLE 2

| Compound of Example No. | $ED_{50}$, mg/kg |
|---|---|
| 2 | $I_{10}$ |
| 6 | 10 |
| 8 | 4.5 |
| 14 | $I_{50}$ |

I = inactive

Toxicity

No toxic effects were observed in these tests.

**Claims**

1. A compound of the formula (I):

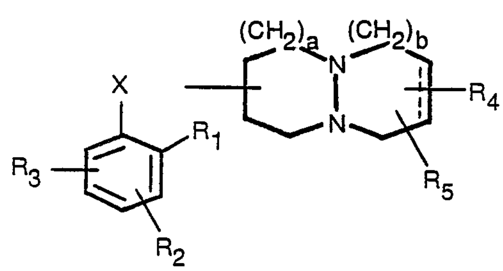

(I)

and pharmaceutically acceptable salts and N-oxides thereof, wherein:

a and b are separately 0, 1 or 2;

X is —CO—NR— wherein R is hydrogen or $C_{1-4}$ alkyl, or —NH—CO—;

$R_1$ is a $C_{1-6}$ alkoxy group;

$R_2$ and $R_3$ are the same or different and are hydrogen, halogen, $CF_3$, $C_{1-7}$ acyl, $C_{1-7}$ acylamino, $C_{1-6}$ alkyl-S(O)$_n$ wherein n is 0, 1 or 2, nitro or amino, $C_{1-6}$ alkoxy, aminocarbonyl or aminosulphonyl optionally substituted by one or two $C_{1-6}$ alkyl groups;

or $R_1$ and $R_2$ taken together on adjacent carbon atoms are methylenedioxy or ethylenedioxy in which case $R_3$ is any one of the groups given for $R_2$ and $R_3$ above;

and either

$R_4$ is hydrogen, $C_{1-6}$ alkyl or phenyl; and $R_5$ is hydrogen; in which case when a and b are 1 and $R_4$ and $R_5$ are both hydrogen, then the dotted line may represent a double bond between the two adjacent carbon atoms (in the $R_4$/$R_5$ substituted ring) opposing the two adjacent nitrogen atoms; or

$R_4$ and $R_5$ are attached to two adjacent carbon atoms and form together with these two carbon atoms a fused benzene ring, which benzene ring may be substituted by $C_{1-6}$ alkyl, $C_{1-6}$ alkoxy, $CF_3$ or halogen;

there being a minimum of two carbon atoms between the amide nitrogen and each of the ring nitrogens.

2. A compound according to claim 1, characterised in that:

X is —CONR— wherein R is as defined in claim I;

$R_2$ and $R_3$ are the same or different and are hydrogen, halogen, $CF_3$, $C_{1-7}$ acyl, $C_{1-7}$ acylamino, $C_{1-6}$ alkyl-S(O)$_n$ wherein n is 0, 1 or 2, nitro or amino, $C_{1-6}$ alkoxy, aminocarbonyl or aminosulphonyl optionally substituted by one or two $C_{1-6}$ alkyl groups; and

$R_4$ and $R_5$ are as defined in claim 1 except that a and b are 1 when the dotted line in formula (I) as defined in claim 1, represents a double bond.

3. A compound according to claim 1, of formula (IV):

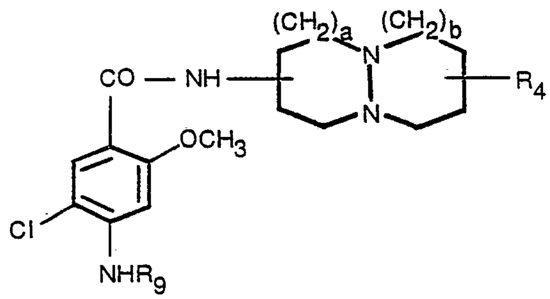

(IV)

wherein a, b and $R_4$ are as defined in claim 1, and $R_9$ is hydrogen or a $C_{1-4}$ alkanoyl group.

4. A compound according to any one of the claims 1 to 3 characterised in that a is 0 and b is 1, a is 1 and b is 0 or a and b are both 1.

5. A compound according to any one of the claims 1 to 4, characterised in that $R_4$ is methyl or hydrogen.

6. A compound according to any one of the claims 2 to 5, characterised in that $R_9$ is hydrogen.

7. (±) 4-Amino-5-chloro-2-methoxy-N-3-{1,6-diazobicyclo [4,4,0]decyl})benzamide,

(±) 4-Amino-5-chloro-2-methoxy-N-(3-{1,5-diazabicyclo[4,3,0]nonyl})benzamide,

(±) 4-Amino-5-chloro-2-methoxy-N-(7-{2-methyl-1,5-diazabicyclo[4,3,0]nonyl})benzamide,

(±) 4-Amino-5-chloro-2-methoxy-N-(8-{2-methyl-1,5-diazabicyclo[4,3,0]nonyl})benzamide, or a pharmaceutically acceptable salt thereof.

8. A process for the preparation of a compound according to claim 1, characterised by reacting a compound of formula (X):

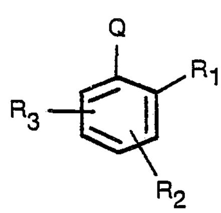

(X)

wherein Q is either COOH or a reactive derivative thereof or $H_2N$, with a compound of the formula (XI):

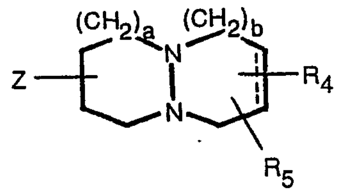

(XI)

wherein Z is HRN when Q is COOH or a reactive derivative thereof, or COOH or a reactive derivative thereof when Q is $H_2N$ and the remaining variables are as defined in claim 1; and thereafter if desired or necessary converting a group $R_2$ or $R_3$ in the thus formed compound to another group $R_2$ or $R_3$ respectively.

9. A pharmaceutical composition comprising a compound according to claim 1 or a pharmaceutically acceptable salt thereof together with a pharmaceutically acceptable carrier.

10. A compound according to claim 1 or a pharmaceutically acceptable salt thereof for use in treating maladies in humans.

**Patentansprüche**

1. Eine Verbindung der Formel (I) und pharmakologisch zulässige Salze und N-Oxide derselben:

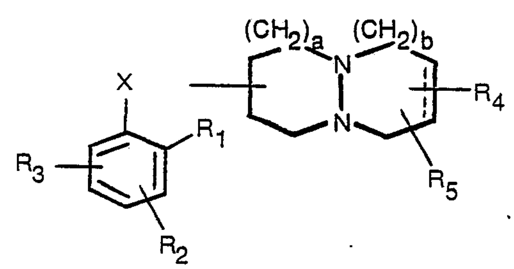

(I)

in welcher

a und b unabhängig voneinander den Wert 0, 1 oder 2 haben;

X die Gruppe —CO—NR—, in welcher R Wasserstoff oder $C_{1-4}$-Alkyl ist, oder die Gruppe —NH—CO— bedeutet:

$R_1$ eine $C_{1-6}$-Alkoxygruppe darstellt;

$R_2$ und $R_3$ gleich oder verschieden sind und jeweils Wasserstoff, Halogen, $CF_3$—, $C_{1-7}$-Acyl, $C_{1-7}$-Acylamino, $C_{1-6}$-Alkyl-S(O)$_n$, wobei n den Wert 0, 1 oder 2 hat, Nitro oder Amino, $C_{1-6}$-Alkoxy, Aminocarbonyl oder Aminosulfonyl, gegebenenfalls durch eine oder zwei $C_{1-6}$-Alkylgruppen substituiert, bedeuten;

oder $R_1$ und $R_2$ zusammen mit (einem) benachbarten Kohlenstoffatom(en) Methylendioxy oder Äthylendioxy bedeuten, in welchem Fall $R_3$ irgendeine der Gruppen darstellt, wie sie vorstehend für $R_2$ und $R_3$ angegeben sind;

und entweder $R_4$ Wasserstoff, $C_{1-6}$-Alkyl oder Phenyl ist und $R_5$ Wasserstoff bedeutet, in welchem Fall, wenn a und b den Wert 1 haben und $R_4$ und $R_5$ beide Wasserstoff sind, die angegebene gestrichelte Linie eine Doppelbindung zwischen den beiden benachbarten Kohlenstoffatomen (in dem $R_4$/$R_5$-substituierten Ring), welche den beiden benachbarten Stickstoffatomen gegenüberstehen, bedeuten kann, oder

$R_4$ und $R_5$ an zwei benachbarte Kohlenstoffatome gebunden sind und zusammen mit diesen beiden Kohlenstoffatomen einen verschmolzenen Benzolring bilden, welcher Benzolring durch $C_{1-6}$-Alkyl, $C_{1-6}$-Alkoxy, $CF_3$ oder Halogen substituiert sein kann, mit der Maßgabe, daß sich mindestens zwei Kohlenstoffatome zwischen dem Amid-Stickstoff und jedem der beiden Ringstickstoffatome befinden.

2. Eine Verbindung gemäß Anspruch 1, dadurch gekennzeichnet, daß

X die Gruppe —CONR— bedeutet, in welcher R wie in Anspruch 1 definiert ist,

$R_2$ und $R_3$ gleich oder verschieden sind und Wasserstoff, Halogen, $CF_3$, $C_{1-7}$-Acyl, $C_{1-7}$-Acylamino, $C_{1-6}$-Alkyl-S(O)$_n$, wobei n den Wert 0, 1 oder 2 hat, Nitro oder Amino, $C_{1-6}$-Alkoxy, Aminocarbonyl oder Aminosulfonyl, welche gegebenenfalls durch ein oder zwei $C_{1-6}$Alkylgruppen substituiert sind, bedeuten und

$R_4$ und $R_5$ wie in Anspruch 1 definiert sind, mit der Maßgabe, daß a und b den Wert 1 haben, falls die gestrichelte Linie in Formel (I), die wie in Anspruch 1 definiert ist, eine Doppelbindung darstellt.

3. Eine Verbindung gemäß Anspruch 1 der Formel (IV):

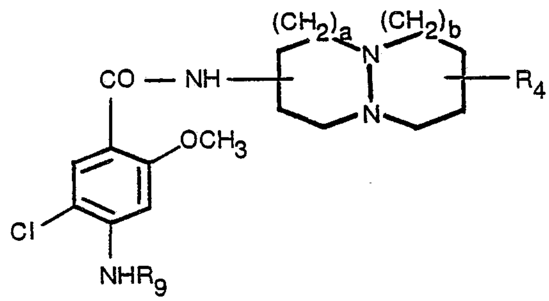

(IV)

in welcher a, b und $R_4$ wie in Anspruch 1 definiert sind und $R_9$ Wasserstoff oder eine $C_{1-4}$Alkanoylgruppe bedeutet.

4. Eine Verbindung gemäß irgendeinem der Ansprüche 1 bis 3, dadurch gekennzeichnet, daß a den Wert Null hat und b den Wert 1 hat, daß a den Wert 1 hat und b den Wert Null hat oder daß a und b beide den Wert 1 haben.

5. Eine Verbindung gemäß irgendeinem der Ansprüche 1 bis 4, dadurch gekennzeichnet, daß $R_4$ Methyl oder Wasserstoff ist.

6. Eine Verbindung gemäß irgendeinem der Ansprüche 2 bis 5, dadurch gekennzeichnet, daß $R_9$ Wasserstoff ist.

7. (±) 4-Amino-5-chlor-2-methoxy-N-(3-[1,6-diazabicyclo[4,4,0]decyl])benzamid,
(±) 4-Amino-5-chlor-2-methoxy-N-(3-[1,5-diazabicyclo[4,3,0]-nonyl])benzamid,
(±) 4-Amino-5-chlor-2-methoxy-N-(7-[2-methyl-1,5-diazabicyclo[4,3,0]nonyl])benzamid,
(±) 4-Amino-5-chlor-2-methoxy-N-(8-[2-methyl-1,5-diazabicyclo[4,3,0]nonyl])benzamid oder ein pharmakologisch zulässiges Salz dieser Verbindungen.

8. Ein Verfahren zur Herstellung einer Verbindung gemäß Anspruch 1, gekennzeichnet durch das Umsetzen einer Verbindung der Formel (X):

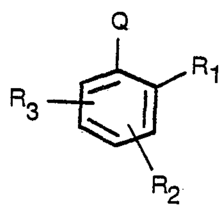

(X)

in welcher Q entweder COOH oder ein reaktives Derivat davon oder $H_2N$ bedeutet, mit einer Verbindung der Formel (XI):

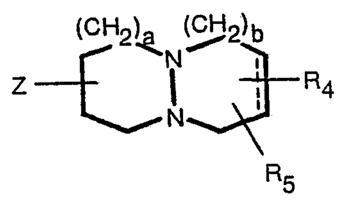

(XI)

in welcher Z die Bedeutung HRN hat, wenn Q COOH oder ein reaktives Derivat davon ist, oder COOH oder ein reaktives Derivat davon darstellt, wenn Q $H_2N$ ist, und die verbleibenden Variablen wie in Anspruch 1 definiert sind, und anschließend, falls gewünscht oder erforderlich, das Umwandeln einer Gruppe $R_2$ oder $R_3$ in der so gebildeten Verbindung in irgendeine andere Gruppe $R_2$ oder $R_3$.

9. Eine pharmazeutische Zusammensetzung, enthaltend eine Verbindung gemäß Anspruch 1 oder ein pharmakologisch zulässiges Salz derselben zusammen mit einem pharmakologisch zulässigen Trägermaterial.

10. Eine Verbindung gemäß Anspruch 1 oder ein pharmakologisch zulässiges Salz derselben zur Anwendung bei der Behandlung von menschlichen Erkrankungen.

**Revendications**

1. Composé représenté par la formule (I)

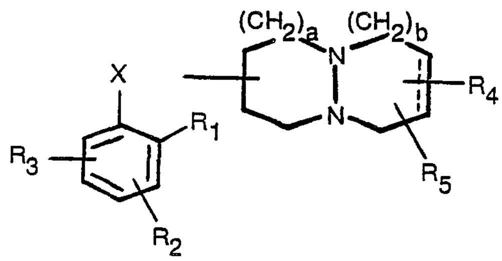

(I)

ainsi que ses sels acceptables du point de vue pharmaceutique et ses N-oxydes, dans laquelle

a et b sont séparément 0, 1 ou 2;

X est un radical —CONR—, où R est un atome d'hydrogène ou un groupe alcoyle en $C_{1-4}$, ou —NH—CO—;

$R_1$ est un groupe alcoxy en $C_{1-6}$;

$R_2$ et $R_3$ sont identiques ou différents et représentent un atome d'hydrogène, d'halogène, un groupe $CF_3$, acyle en $C_{1-7}$, acyl($C_{1-7}$)amino, alcoyl($C_{1-6}$)-S(O)$_n$; où n est 0, 1 ou 2, nitro ou amino, alcoxy en $C_{1-6}$, aminocarbonyle ou aminosulfonyle éventuellement substitué par un ou deux groupes alcoyles en $C_{1-6}$;

ou bien $R_1$ et $R_2$ pris ensemble sur des atomes de carbone adjacents forment un radical méthylènedioxy ou éthylènedioxy, auquel cas $R_3$ est l'un quelconque des groupes indiqués pour $R_2$ et $R_3$ ci-dessus; et soit;

$R_4$ est un atome d'hydrogène, un groupe alcoyle en $C_{1-6}$ ou phényle; et $R_5$ est un atome d'hydrogène; auquel cas, lorsque a et b sont 1 et $R_4$ et $R_5$ sont tous les deux un atome d'hydrogène, la ligne en pointillés peut alors représenter une double liaison entre les deux atomes de carbone adjacents (dans le cycle substitué par $R_4$/$R_5$) opposés aux deux atomes d'azote adjacents;

soit $R_4$ et $R_5$ sont attachés à deux atomes de carbone adjacents et forment ensemble avec ceux-ci un cycle benzène condensé, lequel cycle benzénique peut être substitué par un groupe alcoyle en $C_{1-6}$, alcoxy en $C_{1-6}$, $CF_3$ ou un atome d'halogène;

deux atomes de carbone se trouvant au minimum entre l'atome d'azote du groupe amide et chacun des atomes d'azote du cycle.

2. Composé suivant la revendication 1, caractérisé en ce que:

X est le radical —CONR—, où R est tel que défini dans la revendication 1,

$R_2$ et $R_3$ sont identiques ou différents et représentent un atome d'hydrogène, d'halogène, un groupe $CF_3$, acyle en $C_{1-7}$, acyl($C_{1-7}$)amino, alcoyl($C_{1-6}$)—S(O)$_n$ où n est 0, 1 ou 2, nitro ou amino, alcoxy en $C_{1-6}$, aminocarbonyle ou aminosulfonyle éventuellement substitué par un ou deux groupes alcoyle en $C_{1-6}$; et

$R_4$ et $R_5$ sont tels que définis dans la revendication 1 sauf que a et b sont 1 lorsque la ligne en pointillé dans la formule (I) telle que défini dans la revendication 1, représente une double liaison.

3. Composé suivant la revendication 1, caractérisé en ce qu'il est représenté par la formule (IV) ci-après:

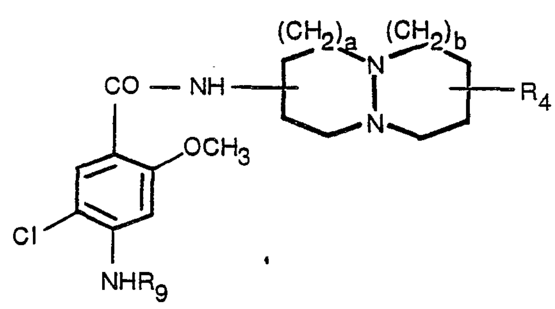

(IV)

dans laquelle a, b et $R_4$ sont tels que définis dans la revendication 1 et $R_9$ est un atome d'hydrogène ou un groupe alcanoyle en $C_{1-4}$.

4. Composé suivant l'une quelconque des revendications 1 à 3, caractérisé en ce que a est 0 et b est 1, a est 1 et b est 0, ou bien a et b sont tous deux 1.

5. Composé suivant l'une quelconque des revendications 1 à 4, caractérisé en ce que $R_4$ est un groupe méthyle ou un atome d'hydrogène.

6. Composé suivant l'une quelconqe des revendications 2 à 5, caractérisé en ce que $R_9$ est un atome d'hydrogène.

7. (±) 4-Amino-5-chloro-2-méthoxy-N-(3-{1,6-diazabicyclo[4,4,0]décyl})benzamide,
(±) 4-Amino-5-chloro-2-méthoxy-N-(3-{1,5-diazabicyclo[4,3,0]nonyl})benzamide,
(±) 4-Amino-5-chloro-2-méthoxy-N-(7-{2-méthyl-1,5-diazabicyclo[4,3,0]nonyl})benzamide,
(±) 4-Amino-5-chloro-2-méthoxy-N-(8-{2-méthyl-1,5-diazabicyclo[4,3,0]nonyl})benzamide, ou un sel acceptable du point de vue pharmaceutique de ceux-ci.

8. Procédé de préparation d'un composé suivant la revendication 1, caractérisé en ce qu'on fait réagir un composé représenté par la formule (X) ci-après:

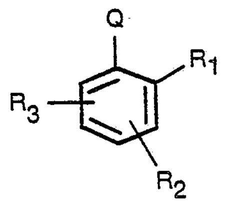

(X)

dans laquelle Q est soit un radical COOH, soit un dérivé réactif de celui-ci, ou $H_2N$, avec un composé représenté par la formule (XI) ci-après:

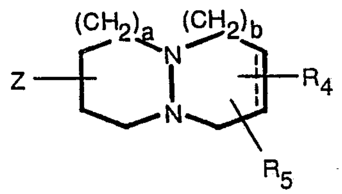

(XI)

dans laquelle Z est HRN lorsque Q est COOH ou un dérivé réactif de celui-ci, ou COOH ou un dérivé réactif de celui-ci lorsque Q est $H_2N$ et les autres variables sont telles que définies dans la revendication 1; et qu'ensuite, si cela est désiré ou nécessaire, on convertit un groupe $R_2$ ou $R_3$ dans le composé de formule (I) ainsi formé un autre groupe $R_2$ ou $R_3$ respectivement.

9. Composition pharmaceutique caractérisée en ce qu'elle comprend un composé suivant la revendication 1 ou un sel acceptable du point de vue pharmaceutique de celui-ci, avec un support acceptable du point de vue pharmaceutique.

10. Composé suivant la revendication 1 ou un sel acceptable du point de vue pharmaceutique de celui-ci, caractérisé en ce qu'il est utile pour traiter des maladies chez l'homme.